# EUROPÄISCHE PATENTANMELDUNG

(11) **EP 2 085 378 A1**
(43) Veröffentlichungstag der Anmeldung: **05.08.2009**
(21) Anmeldenummer: 09006446.0
(22) Anmeldetag: 04.05.2002
(51) Int. Cl.: C07C 303/26, C07C 309/65, C07D 295/02, H01M 6/16, H01M 10/40

(54) **Perfluoralkansulfonsäuresalze**

(30) Priorität: 01.06.2001 DE 10126929; 26.07.2001 DE 10136121
(62) Teilanmeldung aus: 02776502.3
(71) Anmelder: Merck Patent GmbH, 64293 Darmstadt (DE)
(72) Erfinder: Ignatyev, Nikolai, 47058 Duisburg (DE); Schmidt, Michael, 64342 Seeheim-Jugenheim (DE); Heider, Udo, 64560 Riedstadt (DE); Sartori, Peter, 86919 Utting (DE); Kucheryna, Andriy, Kyiv 4215 (UA)

(57) **Zusammenfassung**

Die vorliegende Erfindung betrifft ein Verfahren zur Herstellung von Perfluoralkansulfonsäureestern und deren weitere Umsetzung zu deren Salzen sowie die Verwendung der hergestellten Verbindungen in Elektrolyten und in Batterien, Kondensatoren, Superkondensatoren und galvanischen Zellen.

## Beschreibung

Die vorliegende Erfindung betrifft ein Verfahren zur Herstellung von Verbindungen mit Perfluoralkansulfonsäureresten sowie die Verwendung dieser Verbindungen in Batterien, Kondensatoren, Superkondensatoren und galvanischen Zellen.

Die Verbreitung von tragbaren elektronischen Geräten, wie z.B. Laptop-und Palmtop-Computern, Mobiltelefonen, oder Videokameras und damit auch der Bedarf nach leichten und leistungsfähigen Batterien hat in den letzten Jahren weltweit drastisch zugenommen.

Angesichts dieses sprunghaft gestiegenen Bedarfs nach Batterien und den damit verbundenen ökologischen Problemen kommt der Entwicklung von wiederaufladbaren Batterien mit einer langen Lebensdauer eine stetig wachsende Bedeutung zu.

Lithium-Ionen-Batterien und Doppelschichtkondensatoren mit sehr hohen Kapazitäten (sogenannte Super- oder Ultracapacitors) stellen den derzeitigen Stand der Technik dar. In beiden Systemen werden mit LiPF₆ bzw. N(C₂H₅)₄BF₄ derzeit hydrolyseempfindliche und thermisch instabile Substanzen als Leitsalz verwendet. Im Kontakt mit feuchter Luft bzw. mit Restwasser aus den Lösungsmitteln kann schnell HF entstehen. Neben den toxischen Eigenschaften wirkt HF sehr negativ auf das Zyklenverhalten und somit auf die Performance der elektrochemischen Zellen.

Als Alternative wurden Imide, wie das Bis(trifluormethylsulfonyl)imid oder das Bis(pentafluorethylsulfonyl)imid oder Methanide, wie das Tris(trifluormethylsulfonyl)methanid und deren Derivate vorgestellt.

Aber auch quaternäre Ammonium- und Phosphoniumsalze mit Perfluoralkansulfonat-Anionen wurden als Leitsalze für galvanische Zellen entwickelt. Die Synthese dieser Salze ist jedoch relativ aufwendig, da ein Zwischenprodukt, der Trifluormethansulfonsäuremethylester (Methyltriflat), schwer darzustellen ist.

Methyltriflat ist ein starkes Methylierungsreagenz. Es wird in der präparativen Chemie zur Einführung von Methylgruppen genutzt, z.B. bei der Methylierung von heterocyclischen Verbindungen (Yu, Teylor, Tetrahedron Letter, 1999 (36), 6661-6664) oder der Methylierung von Schwefel-organischen Verbindungen (Tsuge, Hatta, Chem. Letter, 1997 (9), 945-946). Methyltriflat ist wesentlich reaktiver als Methyliodid, Dimethylsulfat und Methyltoluensulfonat, die üblicherweise verwendeten Methylierungsreagenzien bei der Synthese von quaternären Ammonium- und Phosphoniumsalzen.

Es gibt verschiedene Synthesewege für das Methyltriflat (Gramstad, J. Chem. Soc., 1956, 173-180 oder Beard, J. Org. Chem., 1973 (21), 3673-3677). Alle beschriebenen Synthesewege eignen sich nicht für eine Übertragung in einen großen Maßstab, da sie entweder sehr toxische Ausgangsmaterialien, wie z.B. Dimethylsulfat, verwenden, die Ausbeuten sehr niedrig sind, das Reaktionsprodukt aufgereinigt werden muß, oder gefährliche Neben- bzw. Abfallprodukte anfallen, wie z.B. Schwefelsäure die mit Dimethylsulfat verunreinigt ist.

Aufgabe der Erfindung war es daher, ein einfaches Verfahren für die Synthese von Perfluoralkansulfonsäurealkylestern und daraus herstellbare Leitsalze zur Verfügung zu stellen.

Die Aufgabe wird gelöst durch ein Verfahren zur Herstellung von Verbindungen mit Perfluoralkansulfonsäureresten, unter der Verwendung eines Verfahrensschrittes, bei dem Perfluoralkansulfonsäureanhydrid in Gegenwart von Per-fluoralkansulfonsäure mit Dialkylcarbonat zu Perfluoralkansulfonsäurealkylester umgesetzt wird, wobei die Reaktion unter wasserfreier Atmosphäre durchgeführt wird, z.B.:

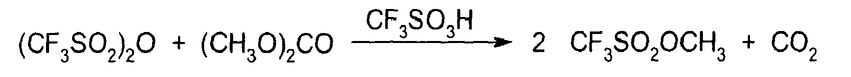

Überraschend wurde gefunden, daß die Umsetzung von Perfluoralkansulfonsäureanhydrid nahezu quantitativ zum Perfluoralkansulfonsäurealkylester erfolgt. Es sind nur katalytische Mengen an Perfluoralkansulfonsäure erforderlich: üblicherweise werden nur 0,01-0,1 mol Perfluoralkansulfonsäure pro 1 mol Perfluoralkansulfonsäureanhydrid benötigt.

Über das erfindungsgemäße Verfahren erhält man Perfluoralkansulfonsäurealkylester, die als Alkylierungsreagenzien verwendet werden können. Sie können zur Alkylierung von heterocyclischen Verbindungen oder Phosphor- und Schwefel-organischen Verbindungen verwendet werden oder zur Darstellung von N-Methylaminosäure mit geringer Racematbildung.

Weiterhin können die erfindungsgemäß erhaltenden Verbindungen mit Perfluoralkansulfonsäureresten auch in galvanischen Zellen, primären Batterien, sekundären Batterien, Kondensatoren und/oder Superkondensatoren beispielsweise als Lösungsmittel eingesetzt werden.

Ferner können die erhaltenen Ester weiter umgesetzt werden zu Perfluoralkansulfonsäuresalzen. Vorteilhafterweise ist es nicht unbedingt erforderlich, dass der erfindungsgemäß zuerst erhaltene Ester isoliert werden muss, da nicht umgesetzte Dialkylcarbonate bei der anschließenden Reaktion mit

XR¹R²R³,

wobei
- X: P oder N ist und
- R¹, R², R³: gleich oder verschieden sind, gegebenenfalls durch eine Einfach- oder Doppelbindung direkt miteinander verbunden sind und jeweils einzeln oder gemeinsam die Bedeutung
- eines Wasserstoffs,
- eines Alkylrestes mit 1 bis 16 C-Atomen, der teilweise mit weiteren Gruppen, vorzugsweise F, Cl, N(CₙF₍₂ₙ₊₁₋ₓ₎Hₓ)₂, O(CₙF₍₂ₙ₋₁₋ₓ₎Hₓ), SO₂(CₙF₍₂ₙ₊₁₋ₓ₎Hₓ), CₙF₍₂ₙ₊₁₋ₓ₎Hₓ mit 1 ≤ n ≤ 6 und 0 ≤ x ≤ 2n+1, ggf. substituiertem Arylrest oder ggf. substituiertem heterocyclischem Rest, substituiert sein kann,
- eines Alkylarylrestes, dessen Alkylengruppe 1 bis 16 C-Atome besitzt und der teilweise mit weiteren Gruppen, vorzugsweise F, Cl, Br, NO₂, CN, Alkyl, Aryl oder heterocyclischem Rest, substituiert sein kann,
- eines Arylrestes, der teilweise mit weiteren Gruppen, vorzugsweise F, Cl, Br, NO₂, CN, Alkyl, Aryl oder heterocyclischem Rest, substituiert sein kann, oder
- eines heterocyclischen Restes, der teilweise mit weiteren Gruppen, vorzugsweise F, Cl, Br, NO₂, CN, Alkyl, Aryl oder heterocyclischem Rest, substituiert sein kann, besitzen,
wobei ein, zwei oder drei CH₂-Gruppen eines Alkyl-oder Alkylenrestes durch gleiche oder verschiedene Heteroatome, vorzugsweise O, NH oder N(Alkyl) mit 1 bis 6 C-Atomen, ersetzt sein können und wobei R¹, R² und R³ nicht gleichzeitig perfluoriert oder perchloriert sein können,

zu den entsprechenden Perfluoralkansulfonsäuresalzen als Lösungsmittel enthalten sein können. Nach der Umsetzung fällt das Perfluoralkansulfonsäuresalz aus. Der nicht umgesetzte Ester muss lediglich abdestilliert werden, während die restliche Perfluoralkansulfonsäure mit XR¹R²R³ neutralisiert ist bzw. für weitere Umsetzungen genutzt werden kann.

In einer bevorzugten Variante des erfindungsgemäßen Verfahrens wird bei dieser nachfolgenden Umsetzung mit dem Ester eine Verbindung XR¹R²R³ eingesetzt, die ausgewählt ist aus der Gruppe

X(C₂H₅)₃, X(C₃H₇)₃, X(C₄H₉)₃,

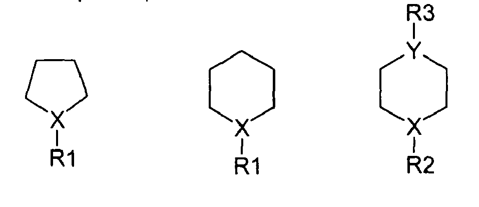

wobei
- X, Y: P oder N ist und
- R¹, R², R³: gegebenenfalls gleich oder verschieden sind, und jeweils einzeln oder gemeinsam die Bedeutung
- eines Wasserstoffes,
- eines Alkylrestes mit 1 bis 16 C-Atomen,
- eines Alkylarylrestes, dessen Alkylengruppe 1 bis 16 C-Atome,
- eines Arylrestes oder
- eines heterocyclischen Restes besitzen,
wobei ein, zwei oder drei CH₂-Gruppen des Rings und/oder der Alkylreste durch gleiche oder verschiedene Heteroatome, vorzugs-weise O, NH oder N(Alkyl) mit 1 bis 6 C-Atomen, ersetzt sein können und
wobei der Ring und/oder der Alkylrest teilweise mit weiteren Gruppen, vorzugsweise mit F, Cl, N(CₙF₍₂ₙ₊₁₋ₓ₎Hₓ)₂, O(CₙF₍₂ₙ₊₁₋ₓ₎Hₓ), SO₂(CₙF₍₂ₙ₊₁₋ₓ₎Hₓ), CₙF₍₂ₙ₊₁₋ₓ₎Hₓ mit 1 ≤ n ≤ 6 und 0 ≤ x ≤ 2n+1, Alkylaryl, Aryl und/oder heterocyclischem Rest, substituiert sein kann und
wobei der Alkylarylrest, der Arylrest und/oder der heterocyclische Rest teilweise mit weiteren Gruppen, vorzugsweise F, Cl, Br, NO₂, CN, Alkyl, Aryl oder heterocyclischem Rest, substituiert sein kann.

Ebenfalls Gegenstand dieser Erfindung sind Perfluoralkansulfonsäuresalze des Typs

Mⁿ⁺ [OSO₂CF₃]ₙ⁻,

worin Mⁿ⁺ (n =1 oder 2) ausgewählt ist aus der folgenden Gruppe: 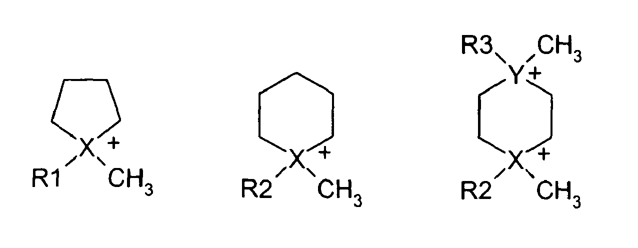 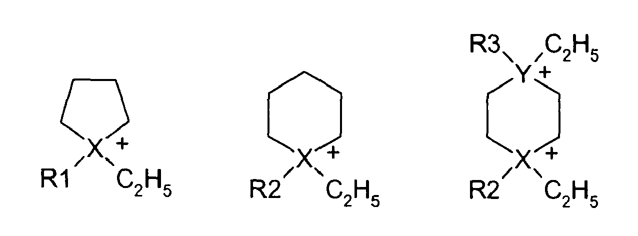 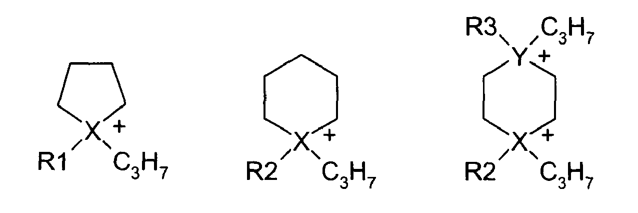 wobei
- X, Y: P oder N ist und
- R¹, R², R³: gegebenenfalls gleich oder verschieden sind, und jeweils einzeln oder gemeinsam die Bedeutung
- eines Wasserstoffes,
- eines Alkylrestes mit 1 bis 16 C-Atomen,
- eines Alkylarylrestes, dessen Alkylengruppe 1 bis 16 C-Atome,
- eines Arylrestes oder
- eines heterocyclischen Restes besitzen,
wobei ein, zwei oder drei CH₂-Gruppen des Rings und/oder der Alkylreste durch gleiche oder verschiedene Heteroatome, vorzugsweise O, NH oder N(Alkyl) mit 1 bis 6 C-Atomen, ersetzt sein können und
wobei der Ring und/oder der Alkylrest teilweise mit weiteren Gruppen, vorzugsweise mit F, Cl, N(CₙF₍₂ₙ₊₁₋ₓ₎Hₓ)₂, O(CₙF₍₂ₙ₊₁₋ₓ₎Hₓ), SO₂(CₙF₍₂ₙ₊₁₋ₓ₎Hₓ), CₙF₍₂ₙ₊₁₋ₓ₎Hₓ mit 1 ≤ n ≤ 6 und 0 ≤ x ≤ 2n+1, Alkylaryl, Aryl und/oder heterocyclischem Rest, substituiert sein kann und
wobei der Alkylarylrest, der Arylrest und/oder der heterocyclische Rest teilweise mit weiteren Gruppen, vorzugsweise F, Cl, Br, NO₂, CN, Alkyl, Aryl oder heterocyclischem Rest, substituiert sein kann.

Diese Perfluoralkansulfonsäuresalze können beispielsweise nach dem erfindungsgemäßen Verfahren hergestellt werden und finden auf vielfältige Weise Anwendung. Neben ihrer Verwendung als Leitsalze z.B. in Elektrolyten können sie auch als Lösungsmittel, insbesondere als ionische Flüssigkeiten, eingesetzt werden. Darüber hinaus besteht auch die Möglichkeit die erfindungsgemäßen Salze in der chemischen Katalyse, insbesondere als Phasentransferkatalysator, zu verwenden. Die Phasentransferkatalyse ist eine Synthesemethode, die für eine Vielzahl von organischen Reaktionen Anwendung findet und die häufig zu hohen Ausbeuten unter vergleichsweise milden Reaktionsbedingungen führt. Bei den meisten phasentransferkatalysierten Reaktionen wird ein Anion aus einer wässerigen oder festen Phase oder einer Grenzfläche mittels des Phasentransferkatalysators in eine organische Phase transportiert, in der es mit erhöhter Reaktivität umgesetzt wird. Die erfindungsgemäßen Perfluoralkansulfonsäuresalze sind daher unter anderem für den Einsatz als ionische Flüssigkeiten oder in der Phasentransferkatalyse geeignet. Ihre diesbezügliche Anwendung bereitet dem Fachmann keinerlei Schwierigkeiten.

In einer weiteren besonders bevorzugten Variante des erfindungsgemäßen Verfahrens können Perfluoralkansulfonsäurersalze hergestellt werden, indem ein erfindungsgemäß erhaltener Ester mit einer Verbindung ausgewählt aus der folgenden Gruppe umgesetzt wird: 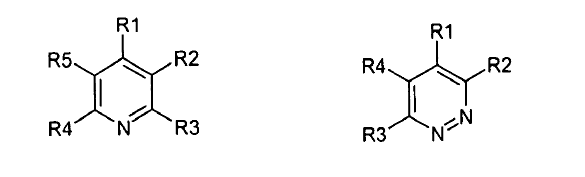 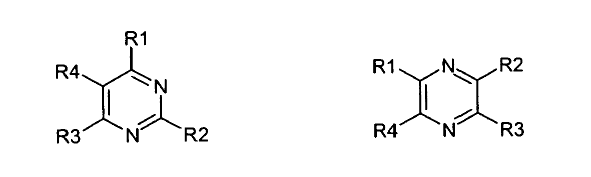 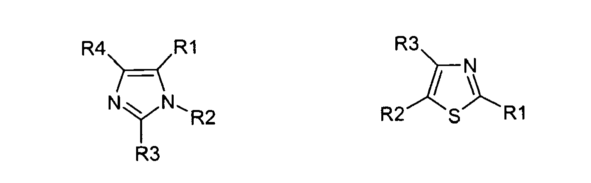 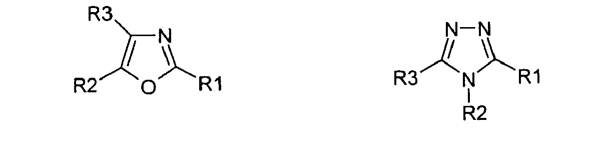 wobei
R¹ bis R⁵ gleich oder verschieden sind, gegebenenfalls durch eine Einfach- oder Doppelbindung direkt miteinander verbunden sind und jeweils einzeln oder gemeinsam die Bedeutung
- eines Wasserstoffs,
- eines Halogens, vorzugsweise Fluor, mit der Maßgabe, dass keine N-Halogen-Bindung vorliegt,
- eines Alkylrests mit 1 bis 8 C-Atomen, der teilweise durch weitere Gruppen, vorzugsweise F, Cl, N(CₙF₍₂ₙ₊₁₋ₓ₎Hₓ)₂, O(CₙF₍₂ₙ₊₁₋ₓ₎Hₓ), SO₂(CₙF₍₂ₙ₊₁₋ₓ₎Hₓ), CₙF₍₂ₙ₊₁₋ₓ₎Hₓ mit 1 ≤ n ≤ 6 und 0 ≤ x ≤ 2n+1, substituiert sein kann,
- eines Arylrestes
- eines Alkylarylrestes
- eines heterocyclischen Restes
- eines alkylheterocyclischen Restes besitzen.

Alle erfindungsgemäß hergestellten Verbindungen mit Perfluoralkansulfonsäureresten, d.h. Perfluoralkansulfonsäureester und insbesondere deren Salze, können in Elektrolyten, galvanischen Zellen, primären und sekundären Batterien, Kondensatoren, Super- bzw. Ultrakondensatoren, beispielsweise als Lösungsmittel oder Leitsalze, eingesetzt werden. Dabei können die Salze sowohl in reiner Form als auch in Form ihrer Mischungen als Leitsalze eingesetzt werden. Es ist auch möglich, die Salze gemeinsam mit weiteren, dem Fachmann bekannten Salzen als Leitsalz zu verwenden.

Die erfindungsgemäß hergestellten Verbindungen mit Perfluoralkansulfonsäureresten, insbesondere die Salze können in flüssigen, gelartigen, polymeren oder festen Elektrolyten verwendet werden. Hierzu können Gemische enthaltend die Leitsalze sowie geeignete Polymeren und/oder geeignete Lösungsmittel eingesetzt werden. Ein Gemisch im Sinne der vorliegenden Erfindung umfasst reine Mischungen der Komponenten, Mischungen, in denen das oder die Salze in einem Polymeren oder Gel eingeschlossen sind sowie Mischungen, in denen zwischen dem oder den Salzen und einem Polymeren oder Gel chemische und/oder physikalische Bindungen bestehen. Im Fall eines gelartigen Elektrolyten enthält das Gemisch vorzugsweise neben dem oder den Salzen und dem Polymer ein geeignetes Lösungsmittel.

Als Lösungsmittel für flüssige oder gelartige Elektrolyte werden besonders bevorzugt aprotische Lösungsmittel oder deren Gemische eingesetzt, die zur Anwendung in einer primären oder sekundären Batterie, einem Kondensator, einem Superkondensator oder einer galvanischen Zelle geeignet sind, zum Beispiel Carbonate, Ester, Ether, Sulfolane oder Nitrile wie beispielsweise Dimethylcarbonat, Diethylcarbonat, Butylencarbonat, Propylencarbonat, Ethylencarbonat, Ethylmethylcarbonat, Methylpropylcarbonat, 1,2-Dimethoxyethan, 1,2-Diethoxyethan, Methylacetat, γ-Butyrolacton, Ethylacetat, Methylpropionat, Ethylpropionat, Methylbutyrat, Ethylbutyrat, Dimethylsulfoxid, Dioxolan, Sulfolan, Acetonitril, Acrylnitril, Tetrahydrofuran, 2-Methyltetrahydrofuran oder deren Gemische.

Als Polymere für polymere oder gelartige Elektrolyte werden vorzugsweise Homopolymere oder Copolymere von Acrylnitril, Vinylidendifluorid, Methyl(meth)acrylat, Tetrahydrofuran, Ethylenoxid, Siloxan, Phosphazen oder eine Mischung aus wenigstens zwei der vorstehend genannten Homopolymeren und/oder Copolymeren eingesetzt, wobei die Polymere zumindest teilweise vernetzt sein können.

Die so erhaltenen Elektrolyte eignen sich zum Einsatz in primären Batterien, sekundären Batterien, Kondensatoren, Super- bzw. Ultrakondensatoren und galvanischen Zellen und stellen ebenfalls einen Gegenstand der vorliegenden Erfindung dar.

Gegenstand der Erfindung sind auch primäre Batterien, sekundäre Batterien, Kondensatoren, Superkondensatoren und galvanische Zellen, die wenigstens ein erfindungsgemäß hergestelltes Perfluoralkansulfonsäuresalz und ggf. weitere Salze und/oder Zusatzstoffe enthalten. Diese weiteren Salze und Zusatzstoffe sind dem Fachmann z.B. aus Doron Aurbach, Nonaqueous Electrochemistry, Marc Dekker Inc., New York 1999; D.Linden, Handbook of Batteries, Second Edition, McGraw-Hill Inc., New York 1995 und G. Mamantov und A.I. Popov, Chemistry of Nonaqueous Solutions, Current Progress, VCH Verlagsgemeinschaft, Weinheim 1994 bekannt.

Die vollständige Offenbarung aller vor- und nachstehend aufgeführten Anmeldungen, Patente und Veröffentlichungen, sowie der korrespondierenden Anmeldungen DE 101 26 929.3 und DE 101 36 121.1, eingereicht am 01.06.2001 und am 26.07.2001, sind durch Bezugnahme in diese Anmeldung eingeführt.

Die im folgenden angeführten Beispiele für den erfindungsgemäßen Gegenstand dienen lediglich der Erläuterung und engen die vorliegende Erfindung keineswegs in irgendeiner Weise ein. Im übrigen ist die beschriebene Erfindung im gesamten beanspruchten Bereich ausführbar.

Alle NMR-Spektren wurden auf einem Bruker Spektrometer WP 80 SY gemessen.

### Beispiele

### Beispiel 1

### Darstellung von Methyltrifluormethansulfonat (Methyltriflat)

In einem 1 l-Rundhalskolben werden 646 g (2,29 mol) Trifluormethansulfonsäureanhydrid und 36 g (0,24 mol) Trifluormethansulfonsäure vorgelegt. Unter ständigem Rühren und Rückflußkühlung werden 206 g (2,29 mol) Dimethylcarbonat bei Raumtemperatur zugegeben. Innerhalb von 10 min erwärmt sich die Lösung auf 50-60°C und wird anschließend noch eine Stunde bei dieser Temperatur weiter gerührt. Anschließend wird die Lösung mit einem Ölbad auf 100-110°C erwärmt und weitere 2 Stunden gerührt. Es werden nach einer Destillation 733 g Methyltrifluormethansulfonat mit einer Reinheit über 99% isoliert (Siedebereich: 98-99°C, Ausbeute: 97,7%).

¹⁹F und ¹H-NMR sind identisch mit den Literaturdaten (Paquette, Encyclopedia of Reagents for Organic Synthesis, 1995, 3617-3622).

Zu dem Rückstand werden 731 g (2,59 mol) Trifluormethansulfonsäureanhydrid und 232 g (2,58 mol) Dimethylcarbonat gegeben und der oben beschriebene Prozeß wiederholt. Es werden 837 g Methyltrifluormethansulfonat mit einer Reinheit über 99% isoliert (Ausbeute: 99,1 %).

Der Prozess kann mehrmals wiederholt werden.

### Beispiel 2

Darstellung von Methylpentafluorethansulfonat In einem 10 ml-Rundhalskolben werden 5,74 g (15,0 mmol) Pentafluorethansulfonsäureanhydrid und 0,31 g (1,55 mmol) Pentafluorethansulfonsäure vorgelegt. Unter ständigem Rühren und Rückflußkühlung werden 1,35 g (15,0 mmol) Dimethylcarbonat bei Raumtemperatur zugegeben. Die Lösung wird eine Stunde bei einer Temperatur von 60°C und anschließend bei 110°C 3 Stunden weiter gerührt. Es werden nach einer Destillation 5,41 g Methylpentafluorethansulfonat isoliert (Siedebereich: 114-115°C, Ausbeute: 84,1 %).

NMR ¹⁹F, ppm: (Lösungsmittel: CDCl₃; Standard: CCl₃F): -80,44 s (CF₃); -115,34 s (CF₂)

NMR ¹H, ppm: (Lösungsmittel: CDCl₃; Standard: TMS): 4,23 s (CH₃)

### Beispiel 3

Darstellung von N-Methyl-N-triethylammoniumtrifluormethansulfonat

Bei Raumtemperatur wird eine Lösung aus 8,35 g (82,7 mmol) Triethylamin in 150 cm³ trockenem Hexan vorgelegt. Unter ständigem Rühren werden innerhalb von 10 min 13,56 g (82,7 mmol) Methyltriflat, hergestellt wie in Beispiel 1, bei Raumtemperatur zugegeben. Die Lösung erwärmt sich und wird eine halbe Stunde weiter gerührt. Dabei wird die Lösung wieder auf Raumtemperatur gebracht. Ein weißer Niederschlag wird abfiltriert und mit Hexan gewaschen. Das Hexan-Filtrat kann für eine weitere Umsetzung genutzt werden. Nach der Trocknung im Vakuum bei 60°C werden 21,81 g eines weißen feinkristallinen Materials werden isoliert (Ausbeute: 99,5%).

NMR ¹⁹F, ppm: (Lösungsmittel: Acetonitril-D₃; Standard: CCl₃F): -78,04 s (CF₃SO₃⁻)

NMR ¹H, ppm: (Lösungsmittel: Acetonitril-D₃; Standard: TMS): 1,25 tm (3CH₃); 2,86 s (CH₃); 3,26 q (3CH₂); J³_{H,H}=7,3 Hz

¹H-NMR-Daten entsprechen den Literaturdaten (R. Weiß, K.-G. Wagner, M. Hertel, Chem. Ber. 117 (1984) S.1965-1972)

### Beispiel 4

Darstellung von Methyl(triethyl)phosphoniumtrifluormethansulfonat

Bei Raumtemperatur wird eine Lösung aus 8,05 g (68,2 mmol) Triethylphosphin in 150 cm³ trockenem Hexan vorgelegt. Unter ständigem Rühren werden innerhalb von 10 min 11,19 g (68,2 mmol) Methyltriflat, hergestellt wie in Beispiel 1, bei Raumtemperatur zugegeben. Die Lösung erwärmt sich und wird eine halbe Stunde weiter gerührt. Dabei wird die Lösung wieder auf Raumtemperatur gebracht. Ein weißer Niederschlag wird abfiltriert und mit Hexan gewaschen. Das Hexan-Filtrat kann für eine weitere Umsetzung genutzt werden. Nach der Trocknung im Vakuum bei 60°C werden 19,02 g eines weißen feinkristallinen Materials isoliert (Schmelzpunkt: 103-104°C, Ausbeute: 98,9%).

NMR ¹⁹F ppm (Lösungsmittel: Aceton-D₆; Standard: CCl₃F): -77,82 s (CF₃SO₃⁻)

NMR ¹H, ppm (Lösungsmittel: Aceton-D₆; Standard: TMS): 1,30 dt (3CH₃); 1,95 d (CH₃); 2,40 dq (3CH₂); J³_{H,H}=7,7 Hz; J²_{P,H}=13,7 Hz; J²_{P,H}=13,8 Hz; J³_{P,H}=18,8 Hz

Elementaranalyse:
gefunden: 33,72% C, 6,57% H, 11,14% S
berechnet: 34,04% C, 6,43% H, 11,36% S ((C₂H₅)₃PCH₃⁺ CF₃SO₃⁻)

### Beispiel 5

### Darstellung von 1,3-Dimethylimidazoliumtrifluormethansulfonat

Zu 6,67 g (81,2 mmol) 1-Methylimidazol werden in einem Rundkolben unter Rühren und Eisbad-Kühlung 13,70 g (83,5 mmol) Methyltriflat innerhalb von 10 Minuten zugetropft. Das Reaktionsgemisch erwärmt sich und wird unter Rückflußkühlung 1 Stunde bei 70-75°C gerührt. Der Überschuss an Methyltriflat wird im Vakuum bei 60°C entfernt. 20,00 g eines weißen Pulvers werden isoliert.

NMR ¹⁹F, ppm (Lösungsmittel: Acetonitril-D₃; Standard: CCl₃F): -78,14 s (CF₃SO₃⁻)

NMR ¹H, ppm (Lösungsmittel: Acetonitril-D₃; Standard: TMS): 3,82 s (2CH₃); 7,35 d (2H); 8,53 t (1H); J⁴_{H;H}=1,6 Hz

¹H-NMR-Daten entsprechen den Literaturdaten (U. Zollner, Tetrahedron, 44, No.24 (1988), S.7413-7426)

### Beispiel 6

Darstellung von N,N-Dimethylpyrrolidiniumtrifluormethansulfonat

Bei Raumtemperatur wird eine Lösung aus 6,83g (80,2mmol) N-Methylpyrrolidin in 150 cm³ trockenem Hexan vorgelegt. Unter ständigem Rühren werden innerhalb von 10min 13,12g (80,2mmol) Methyltriflat, hergestellt wie in Beispiel 1, bei Raumtemperatur zugegeben, wobei sich die Lösung erwärmt. Nach einer halben Stunde wird die Lösung wieder auf Raumtemperatur gebracht. Der aufgefallene weiße Niederschlag wird abfiltriert mit Hexan gewaschen und im Vakuum bei 60°C getrocknet. Es werden 16,61g eines weißen feinkristallinen Materials isoliert (Schmelzpunkt (mit Zersetzung): 308-310°C, Ausbeute 98,3%).

NMR ¹⁹F, ppm (Lösungsmittel: Acetonitril-D₃; Standard: CCl₃F): -78,00 s (CF₃SO₃⁻)

NMR ¹H, ppm (Lösungsmittel: Acetonitril-D₃; Standard: TMS): 2,17 m (2H); 3,07 s (CH₃); 3,45 m (2H)

Elementaranalyse nach Umkristallisation aus Methanol:
gefunden: 33,66% C, 5,68% H, 5,60% N, 13,06% S
berechnet: 33,73% C, 5,66% H, 5,62% N, 12,86% S (C₇H₁₄ F₃NO₃S)

### Beispiel 7

Darstellung von N,N-Dimethylpiperidiniumtrifluormethansulfonat

Bei Raumtemperatur wird eine Lösung aus 7,76g (78,2mmol) N-Methylpiperidin in 150 cm³ trockenem Hexan vorgelegt. Unter ständigem Rühren werden innerhalb von 10min 12,83g (78,2mmol) Methyltriflat, hergestellt wie in Beispiel 1, bei Raumtemperatur zugegeben, wobei sich die Lösung erwärmt. Nach einer halben Stunde wird die Lösung wieder auf Raumtemperatur gebracht. Der aufgefallene weiße Niederschlag wird abfiltriert mit Hexan gewaschen und im Vakuum bei 80°C getrocknet. Es werden 19,72g eines weißen feinkristallinen Materials isoliert (Schmelzpunkt nach Umkristallisation aus Methanol: 255-256°C, Ausbeute 95,8%).

NMR ¹⁹F, ppm (Lösungsmittel: Acetonitril-D₃; Standard: CCl₃F): -78,06 s (CF₃SO₃⁻) NMR ¹H, ppm (Lösungsmittel: Acetonitril-D₃; Standard: TMS): 1,79 m (3CH₂); 3,02 s (2CH₃); 3,27 m (2CH₂)

Elementaranalyse nach Umkristallisation aus Methanol:
gefunden: 36,44% C, 6,07% H, 5,31% N, 12,20% S
berechnet: 36,50% C, 6,13% H, 5,32% N, 12,18% S (C₈H₁₆ F₃NO₃S)

## Patentansprüche

1. Verbindungen des Typs
Mⁿ⁺ [OSO₂CF₃]ₙ⁻,
worin Mⁿ⁺ (n =1 oder 2) ausgewählt ist aus der folgenden Gruppe: 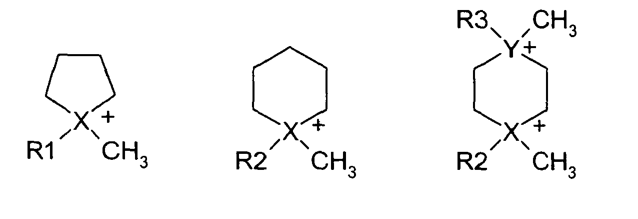 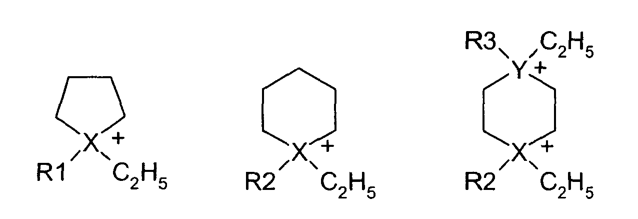 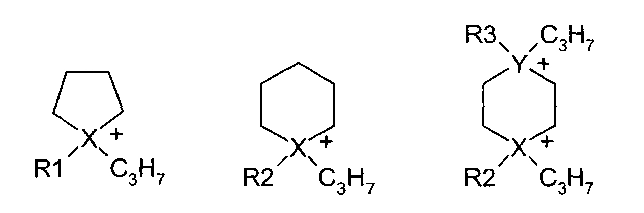 wobei
X, Y P oder N ist und
R¹, R², R³ gegebenenfalls gleich oder verschieden sind, und jeweils einzeln oder gemeinsam die Bedeutung
- eines Wasserstoffes,
- eines Alkylrestes mit 1 bis 16 C-Atomen,
- eines Alkylarylrestes, dessen Alkylengruppe 1 bis 16 C-Atome,
- eines Arylrestes oder
- eines heterocyclischen Restes besitzen,
wobei ein, zwei oder drei CH₂-Gruppen des Rings und/oder der Alkylreste durch gleiche oder verschiedene Heteroatome, vorzugsweise O, NH oder N(Alkyl) mit 1 bis 6 C-Atomen, ersetzt sein können und
wobei der Ring und/oder der Alkylrest teilweise mit weiteren Gruppen, vorzugsweise mit F, Cl, N(CₙF₍₂ₙ₊₁₋ₓ₎Hₓ)₂,
O(CₙF₍₂ₙ₊₁₋ₓ₎Hₓ), SO₂(CₙF₍₂ₙ₊₁₋ₓ)Hₓ), CₙF₍₂ₙ₊₁₋ₓ₎Hₓ mit 1 ≤ n ≤ 6 und 0 ≤ x ≤ 2n+1, Alkylaryl, Aryl und/oder heterocyclischem Rest, substituiert sein können und
wobei der Alkylarylrest, der Arylrest und/oder der heterocyclische Rest teilweise mit weiteren Gruppen, vorzugsweise F, Cl, Br, NO₂, CN, Alkyl, Aryl oder heterocyclischem Rest, substituiert sein kann.

2. Verwendung einer oder mehrerer Verbindungen nach Anspruch 1 in Elektrolyten allein oder in Mischung mit anderen Salzen.

3. Verwendung ein oder mehrerer Verbindungen nach Anspruch 1 als Leitsalz in primären Batterien, sekundären Batterien, Kondensatoren, Superkondensatoren oder galvanischen Zellen, ggf. auch in Kombination mit weiteren Salzen.

4. Flüssiger, gelartiger, polymerer oder fester Elektrolyt, enthaltend eine oder mehrere Verbindungen nach Anspruch 1 allein oder in Mischung mit anderen Salzen.

5. Primäre Batterien, sekundäre Batterien, Kondensatoren, Superkondensatoren und/oder galvanischen Zellen enthaltend eine oder mehrere Verbindungen nach Anspruch 1 oder enthaltend Elektrolyte nach Anspruch 4.

6. Verwendung einer oder mehrerer Verbindungen nach Anspruch 1 als Lösungsmittel.

7. Verwendung einer oder mehrerer Verbindungen nach Anspruch 1 als Katalysator, insbesondere als Phasentransferkatalysator.

8. Verbindungen nach Anspruch 1, **dadurch gekennzeichnet, dass** R¹, R², R³ gegebenenfalls gleich oder verschieden sind, und jeweils einzeln oder gemeinsam die Bedeutung
- eines Wasserstoffes,
- eines Alkylrestes mit 1 bis 16 C-Atomen,
- eines Alkylarylrestes, dessen Alkylengruppe 1 bis 16 C-Atome,
- eines Arylrestes oder
- eines heterocyclischen Restes besitzen.

9. Verbindungen nach Anspruch 1 N,N-Dimethylpyrrolidiniumtrifluormethansulfonat N,N-Dimethylpiperidiniumtrifluormethansulfonat.
